# EUROPÄISCHE PATENTSCHRIFT

(11) **EP 4 505 959 B1**
(45) Veröffentlichungstag und Bekanntmachung des Hinweises auf die Patenterteilung: **13.05.2026**
(21) Anmeldenummer: 24192817.5
(22) Anmeldetag: 05.08.2024
(51) Int. Cl.: A61B 18/20

(54) **MIKROMANIPULATOR ZUR MANIPULATION EINES LASERSTRAHLS**
MICROMANIPULATOR FOR MANIPULATING A LASER BEAM
MICROMANIPULATEUR POUR LA MANIPULATION D'UN FAISCEAU LASER

(30) Priorität: 10.08.2023 DE 202023104535 U
(43) Veröffentlichungstag der Anmeldung: 12.02.2025
(73) Patentinhaber: Karl Leibinger Asset Management GmbH & Co. KG, 78570 Mühlheim an der Donau (DE)
(72) Erfinder: SIMON, Philipp, 79114 Freiburg (DE); HEINZ, Oliver, 79104 Freiburg (DE); GLOTZ, Manfred, 79112 Freiburg (DE)
(74) Vertreter: Lorenz Seidler Gossel Part. mbB

(56) Entgegenhaltungen:
- EP-B1- 0 069 987
- WO-A1-2017/139853
- US-A- 4 091 814
- US-A- 4 526 447
- US-A- 5 257 992
- US-A1- 2022 104 877

## Beschreibung

Die vorliegende Erfindung betrifft einen Mikromanipulator zur Manipulation eines Laserstrahls eines Laserchirurgie-Geräts, mit einem Scanner zur Erzeugung mindestens einer Scanfigur und mindestens einem Bedienelement zur Ansteuerung des Scanners.

In der medizinischen Anwendung von Lasergeräten haben sich Laserchirurgie-Geräte, beispielsweise auf Basis von CO₂-Lasern oder Festkörper- und Dioden-Lasern etabliert.

Für die mikrochirurgischen Anwendungen wird die Beobachtung der Operationen über Operationsmikroskope vorgenommen, um auch sehr kleine Operationen adäquat durchführen zu können.

Bei der Anwendung von Laserlicht für die chirurgischen Eingriffe kann dieses über einen Mikromanipulator in den Beobachtungsstrahlengang des Operationsmikroskops eingespiegelt werden. Hierzu wird der Mikromanipulator als eigenständiges Gerät unter das Operationsmikroskop montiert. Ein großer, wellenlängenabhängiger Spiegel im Mikromanipulator befindet sich dadurch direkt unter dem Objektiv des Operationsmikroskops. Der Spiegel ist durchlässig für die OP-Feldbeleuchtung und für die Beobachtung und hochreflektierend für das Laserlicht. Er steht unter ca. 45° vor dem Objektiv und reflektiert so den Laserstrahl direkt in das Operationsfeld.

In Kombination mit dem Mikromanipulator dient ein Scanner, welcher sich am optischen Eingang des Mikromanipulators befindet, dazu, den Laserstrahl in x - und y - Richtung zu bewegen. Der Scanner umfasst insbesondere eine elektronisch gesteuerte Spiegeleinheit, über welche der Laserstrahl bewegt wird. Durch die schnelle Strahlbewegung entsteht eine Strahlablenkungsstruktur, hier als Scanfigur bezeichnet, auf dem Operationsfeld.

Wird als Scanfigur eine Linie gewählt, so besitzt diese Linie eine Orientierung im Operationsfeld. Eine Linie wird verwendet, wenn die Operation, d.h. die Anwendung des Laserlichts, einen Schnitt erzeugen soll. Durch die elektronische Steuerung des Scanners erfolgt der Schnitt sehr gleichmäßig über die Linienlänge. Dieser Vorteil gegenüber einer handgeführten Linie wird in der Laseroperationstechnik zum präzisen Schneiden verwendet.

Stimmt die Orientierung der Linie nicht mit der gewünschten Schnittlinie überein, so muss die Scanfigur gedreht werden.

Bestehende Mikromanipulatoren mit Scanner besitzen daher bereits die Möglichkeit, Scanfiguren zu drehen. Bei den bekannten Systemen wird eine Scanfigur mit einer festen Drehgeschwindigkeit links- oder rechtsherum gedreht, wenn ein Bedienelement für Linksdrehung oder ein Bedienelement für Rechtsdrehung betätigt wird. Auch ein Kombielement mit beiden Aktivierungsmöglichkeiten ist bekannt.

Bei dieser bekannten Technik kann die Drehgeschwindigkeit der Scanfigur nicht verändert werden; nur die Drehrichtung. Von Nachteil ist, dass das Bedienelement so lange betätigt werden muss, bis die Drehung der Scanfigur die gewünschte Orientierung erreicht hat. Hierbei tritt die Schwierigkeit auf, dass die Drehung zu langsam erfolgt und die Operationszeit verlängert. Oder die Drehung erfolgt zu schnell und die Drehung geht über die angestrebte Orientierung hinaus. Dadurch ist es notwendig, in entgegengesetzter Drehrichtung "zurückzudrehen". Dies kann einen iterativen Prozess auslösen, bis die gewünschte Orientierung der Drehfigur erreicht ist.

Das Drehen einer Scanfigur kann hierbei zum einen über Funktionstasten am Lasergerät bewirkt werden. Hierzu muss der Anwender vom Patient auf den Laser wechseln oder eine zweite Person per Sprache zur Drehung der Figur anweisen. Eine Steuerung vom Anwendungsteil, insbesondere vom OP-Mikroskop, ist über den Positionierhandgriff (Joystick) am Mikromanipulator, mit dem die Scanfigur positioniert wird, praktikabler. Hierbei bestehen die bisherigen Umsetzungen in Tasten für Rechts-Links-Drehung in Form von Drucktasten oder Drehtastern. Hierdurch werden jedoch mechanische Kräfte auf den Joystick ausgeübt, die leicht eine De-Positionierung der Scanfigur bewirken können. Ebenso sind Tastungen an Schrittweiten und Dauertastung wie oben beschrieben an Fortschrittsgeschwindigkeiten gebunden.

Die WO 2017/139 853 A1 beschreibt eine Steuervorrichtung für eine Spaltlampenanordnung mit einem Laser zur Erzeugung eines Behandlungsfleckmusters. Die Steuervorrichtung umfasst einen Joystick mit einem unteren Teil und einem oberen Teil. Zwischen dem oberen und unteren Teil ist ein drehbarer Ring angeordnet, der zur Rotation des Behandlungsfleckmusters eingerichtet ist. Der drehbare Ring lässt sich um wenige Grad drehen, und weist zwei Stufen auf, welche eine langsame und eine schnelle Rotation des Behandlungsfleckmusters ermöglichen.

Aufgabe der vorliegenden Erfindung ist es daher, einen verbesserten Mikromanipulator zur Manipulation eines Laserstrahls eines Laserchirurgie-Geräts zur Verfügung zu stellen.

Diese Aufgabe wird durch einen Mikromanipulator gemäß Anspruch 1 gelöst. Bevorzugte Ausgestaltungen der vorliegenden Erfindung sind Gegenstand der Unteransprüche.

Die vorliegende Erfindung umfasst einen Mikromanipulator zur Manipulation eines Laserstrahls eines Laserchirurgie-Geräts, mit einem Scanner zur Erzeugung mindestens einer Scanfigur und mindestens einem Bedienelement zur Ansteuerung des Scanners. Das Bedienelement umfasst erfindungsgemäß ein Drehelement, wobei eine Drehung des Drehelements eine Drehung der Scanfigur bewirkt, welche von der Größe und/oder Geschwindigkeit der Drehung des Drehelements abhängt.

Die Scanfigur dreht sich daher entsprechend der Drehung des Drehelements. Dies ermöglicht eine gezielte Drehung der Scanfigur in eine gewünschte Position, welche zumindest einige und bevorzugt alle der oben genannten Nachteile vermeidet. Das Drehelement ist ohne Begrenzung des Drehwinkels frei drehbar, d.h. der Drehwinkel ist nicht limitiert und das Drehelement kann um beliebig viele Umdrehungen gedreht werden. Die Scanfigur folgt dieser freien Drehung des Drehelements ohne Einschränkungen, d.h. es sind beliebig viele Rotationen der Scanfigur möglich.

Bei der Größe der Drehung des Drehelements handelt es sich beispielsweise um den Betrag der durch die Drehung erzeugten Drehwinkeldifferenz zwischen einer Ausgangsstellung und einer aktuellen Stellung des Drehelements, oder um die durch die Drehung aktuell eingenommene absolute Drehwinkelposition des Drehelements. Bei der Geschwindigkeit der Drehung des Drehelements handelt es sich insbesondere um die aktuelle Veränderungsrate des Drehwinkels des Drehelements bei der Drehung.

Der Scanner umfasst bevorzugt eine elektronisch gesteuerte Spiegeleinheit, über welche der Laserstrahl bewegt wird. Durch die schnelle Strahlbewegung entsteht eine Strahlablenkungsstruktur, hier als Scanfigur bezeichnet, auf dem Operationsfeld.

Gemäß einer möglichen Ausgestaltung der vorliegenden Erfindung umfasst der Mikromanipulator einen schwenkbar an einer Basis angeordneten Joystick, über welchen die Position der Scanfigur einstellbar ist.

Gemäß einer ersten möglichen Ausgestaltung umfasst der Mikromanipulator einen Spiegel, über welchen der Laserstrahl in das Operationsfeld eingekoppelt werden kann.

Bei dem Spiegel handelt es sich bevorzugt um einen wellenlängenabhängigen Spiegel, welcher bei Montage des Mikromanipulators an einem Operationsmikroskop bevorzugt unter dem Objektiv des Operationsmikroskops platziert ist. Der Spiegel ist durchlässig für die OP-Feldbeleuchtung und für die Beobachtung und reflektierend für das Laserlicht. Er ist bevorzugt so angeordnet, dass er den Laserstrahl direkt in das Operationsfeld reflektiert.

Gemäß einer ersten möglichen Ausgestaltung der vorliegenden Erfindung ist die Ausrichtung des Spiegels durch den Joystick veränderbar ist, um die Position der Scanfigur einzustellen.

Insbesondere kann der Spiegel bevorzugt so mittels des Joysticks gekippt werden, dass der reflektierte Laserstrahl in x- und y- Richtung bewegt wird.

Der Joystick kann hierbei entweder mechanisch mit dem Spiegel verbunden sein und diesen mechanisch bewegen, oder Mikroaktoren zur Bewegung des Spiegels ansteuern.

Gemäß einer zweiten möglichen Ausgestaltung der vorliegenden Erfindung erfolgt die Einstellung der Position der Scanfigur dagegen durch eine Ansteuerung des Nullpunkts des separat zum Spiegel vorgesehenen Scanners mittels des Joysticks. In diesem Fall kann der Spiegel starr an dem Mikromanipulator angeordnet sein.

Der Scanner ist bevorzugt zwischen dem optischen Eingang des Mikromanipulators und dem Spiegel angeordnet. Durch die Bewegung des Spiegels kann daher die Position der Scanfigur verändert werden.

Ist der Spiegel über Mikroaktoren bewegbar, können diese jedoch auch zur Erzeugung der Scanfigur eingesetzt werden, so dass der Spiegel gleichzeitig als Spiegel des Scanners arbeitet und kein separater Scanner benötigt wird.

Gemäß einer möglichen Ausgestaltung der vorliegenden Erfindung ist der Joystick an einer die optischen Komponenten des Mikromanipulators enthaltenden Baueinheit angeordnet.

Wird ein elektronischer Joystick eingesetzt, kann dieser jedoch auch als eine separate Baueinheit ausgeführt werden, welche lediglich in Signalverbindung zu der die optischen Komponenten des Mikromanipulators enthaltenden Baueinheit steht. Die Signalverbindung kann kabelgebunden oder kabellos erfolgen.

**In** einer mögliche Ausgestaltung befindet sich der Joystick hierbei auf einer separaten Konsole, die vom Anwender frei positionierbar ist. Die Übertragung der Steuerungssignale vom Joystick an die Steuerung des Mikromanipulators, insbesondere an die Steuerung der Mikroaktoren und/oder des Scanners erfolgt in einer ersten Ausführungsform über ein Signalkabel, in einer weiteren Ausführungsform über Funksignale kabellos.

Gemäß einer möglichen Ausgestaltung der vorliegenden Erfindung ist das Drehelement an dem Joystick angeordnet. Hierdurch können die Position und die Ausrichtung der Scanfigur beide über den Joystick angesteuert werden
Gemäß einer möglichen Ausgestaltung der vorliegenden Erfindung ist das Drehelement um eine Hauptachse des Joysticks drehbar.

Gemäß einer möglichen Ausgestaltung der vorliegenden Erfindung ist das Drehelement im Bereich der Spitze des Joysticks angeordnet und wird bevorzugt durch eine drehbare Spitze des Joysticks gebildet.

Gemäß einer möglichen Ausgestaltung der vorliegenden Erfindung umfasst das Bedienelement einen Sensor, durch welchen eine absolute Drehwinkelposition und/oder eine Größe und/oder Geschwindigkeit einer relativen Veränderung der Drehwinkelposition des Drehelements erfasst wird.

Gemäß einer möglichen Ausgestaltung der vorliegenden Erfindung ist vorgesehen, dass der Sensor die erfasste Größe als einen analogen oder digitalen Wert an eine Steuerung des Scanners übermittelt.

Gemäß einer möglichen Ausgestaltung der vorliegenden Erfindung ist das Drehelement stufenlos oder gestuft in eine Mehrzahl von Drehwinkelpositionen drehbar.

Gemäß einer möglichen Ausgestaltung der vorliegenden Erfindung ist das Drehelement in zwei entgegengesetzte Drehrichtungen drehbar, wobei die Drehung am Drehelement eine gleichsinnige Drehung der Scanfigur bewirkt. Die Scanfigur kann daher je nach Richtung der Drehung am Drehelement in entgegengesetzte Richtungen gedreht werden.

Gemäß einer möglichen Ausgestaltung der vorliegenden Erfindung ist vorgesehen, dass die Drehung des Drehelements eine Drehung der Scanfigur bewirkt, welche von der Größe der Drehung des Drehelements abhängt. Insbesondere hängt hierbei der Betrag der durch die Drehung erzeugten Drehwinkeldifferenz zwischen einer Ausgangsstellung und einer aktuellen Stellung der Scanfigur von einem Betrag der durch die Drehung erzeugten Drehwinkeldifferenz zwischen einer Ausgangsstellung und einer aktuellen Stellung des Drehelements ab.

Gemäß einer möglichen Ausgestaltung der vorliegenden Erfindung ist vorgesehen, dass die Drehung des Drehelements eine zur Größe der Drehung proportionale Drehung der durch den Scanner erzeugten Scanfigur bewirkt, d.h. die Größe der Drehung der Scanfigur ist eine monoton steigende Funktion der Größe der Drehung des Drehelementes, insbesondere eine streng monoton steigende Funktion.

Gemäß einer möglichen Ausgestaltung der vorliegenden Erfindung ist vorgesehen, dass zumindest in einem Bedienmodus die Größe der Drehung der Scanfigur ein festes Verhältnis zur Größe der Drehung des Drehelements besitzt.

Gemäß einer möglichen Ausgestaltung der vorliegenden Erfindung ist vorgesehen, dass das feste Verhältnis einstellbar ist.

Gemäß einer möglichen Ausgestaltung der vorliegenden Erfindung ist vorgesehen, dass zumindest in einem Bedienmodus die Größe der Drehung der Scanfigur ein Verhältnis zur Größe der Drehung des Drehelements besitzt, welches von der Drehgeschwindigkeit der Drehung des Drehelements abhängt.

Bevorzugt erhöht sich hierbei die Größe der Drehung der Scanfigur im Verhältnis zur Größe der Drehung des Drehelements bei steigender Drehgeschwindigkeit der Drehung des Drehelements.

Gemäß einer möglichen Ausgestaltung der vorliegenden Erfindung kann zwischen den beiden oben beschriebenen Betriebsmodi umgeschaltet werden.

Gemäß einer möglichen Ausgestaltung der vorliegenden Erfindung umfasst das Bedienelement ein Druck- oder Zugeingabeelement, wobei durch Betätigen des Druck- oder Zugeingabeelements mindestens ein Parameter des Mikromanipulators veränderbar ist. Bei diesem Parameter handelt es sich um einen zusätzlich zur Ausrichtung der Scanfigur einstellbaren Parameter.

**In** einer bevorzugten Ausgestaltung ist das Drehelement als ein solches Druck- oder Zugeingabeelement ausgestaltet oder weist ein solches Druck- oder Zugeingabeelement auf.

Beispielsweise kann das Drehelement in axialer Richtung beweglich sein, wobei ein Sensor die Bewegung erfasst, wobei aufgrund der Signale des Sensors der mindestens eine Parameter des Mikromanipulators verändert wird. Insbesondere kann das Drehelement am Joystick in axialer Richtung beweglich angeordnet sein.

Alternativ kann an der Spitze des Joysticks ein Druck- oder Zugeingabeelement vorgesehen sein, welches von dem Drehelement umgeben ist.

Insbesondere kann es sich bei dem Druck- oder Zugeingabeelement um einen Druck- oder Zugtaster oder Druck- oder Zugschalter handeln.

Gemäß einer ersten möglichen Variante der vorliegenden Erfindung kann durch Betätigen des Druck- oder Zugeingabeelement unmittelbar zwischen unterschiedlichen Parameter-Einstellungen eines Parameters gewechselt werden. Insbesondere werden die Parameter-Einstellungen durch die Anzahl der Betätigungen oder die Dauer der Betätigung gewechselt.

Gemäß einer zweiten möglichen Variante der vorliegenden Erfindung kann durch Betätigen des Druck- oder Zugeingabeelement in einen Betriebsmodus gewechselt werden, in welchen dem Drehelement eine andere Steuerungsfunktionen zukommt und durch Drehen des Drehelements der Parameter des Mikromanipulators veränderbar ist. Weiterhin können auch mehrere Betriebsmodi vorgesehen sein, in welchen das Drehelement jeweils zur Veränderung unterschiedlicher Parameter eingesetzt wird, vorgesehen sein, wobei zwischen den Betriebsmodi durch Betätigen des Druck- oder Zugeingabeelements gewechselt wird.

Die beiden Varianten können auch in Kombination implementiert sein. Beispielsweise kann durch ein Betätigen mit einer ersten Dauer unmittelbar zwischen Parameter-Einstellungen eines Parameters gewechselt werden und durch Betätigen mit einer zweiten, insbesondere längeren Dauer zwischen Betriebsmodi mit einer geänderten Funktion des Drehelements umgeschaltet werden.

Für jeden der im folgenden beschriebenen möglichen Parameter kann die Ansteuerung des Parameters durch eine der beiden oben beschriebenen Varianten implementiert sein.

Gemäß einer möglichen Ausgestaltung der vorliegenden Erfindung ist vorgesehen, dass ein durch das Betätigen des Druck- oder Zugeingabeelements veränderbarer Parameter die Art und/oder Größe der Scanfigur ist.

Beispielsweise kann daher durch Drücken oder Ziehen des Druck- oder Zugeingabeelements zwischen unterschiedlichen Mustern umgeschaltet werden, beispielsweise einem linienförmigen Muster und mindestens einem oder mehreren weiteren Mustern wie einem Kreis, Quadrat, Rechteck, Dreieck oder Sechseck. Alternativ oder zusätzlich kann durch Drücken oder Ziehen des Druck- oder Zugeingabeelements die Größe des gewählten Musters verändert werden, beispielsweise zwischen unterschiedlichen Größen umgeschaltet werden.

Gemäß einer möglichen Ausgestaltung der vorliegenden Erfindung ist vorgesehen, dass ein durch das Betätigen des Druck- oder Zugeingabeelements veränderbarer Parameter ein Geometrieparameter der Form einer gewählten Scanfigur ist, insbesondere die Krümmung einer linienförmigen Scanfigur. Beispielsweise kann daher durch Drücken oder Ziehen des Druck- oder Zugeingabeelements zwischen unterschiedlichen Krümmungen umgeschaltet oder durch Drücken oder Ziehen des Druck- oder Zugeingabeelements eine kontinuierliche Veränderung der Krümmung vorgenommen werden.

Gemäß einer möglichen Ausgestaltung der vorliegenden Erfindung ist vorgesehen, dass ein durch das Betätigen des Druck- oder Zugeingabeelements veränderbarer Parameter die Laserleistung und/oder die Scanfigurengeschwindigkeit ist.

Gemäß einer möglichen Ausgestaltung der vorliegenden Erfindung ist vorgesehen, dass ein durch das Betätigen des Druck- oder Zugeingabeelements veränderbarer Parameter der Bedienmodus des Drehelements ist. Insbesondere kann das Umschalten zwischen den oben genannten Modi durch diese Betätigung erfolgen.

Gemäß einer möglichen Ausgestaltung der vorliegenden Erfindung ist vorgesehen, dass ein durch das Betätigen des Druck- oder Zugeingabeelements veränderbarer Parameter das Verhältnis zwischen der Größe der Drehung der Scanfigur und der Größe der Drehung des Drehelements ist. Insbesondere kann daher die Einstellung dieses oben beschriebenen Verhältnisses über das Betätigen des Druck- oder Zugeingabeelements erfolgen.

Beispielsweise kann daher durch Drücken oder Ziehen des Druck- oder Zugeingabeelements zwischen unterschiedlichen Werten der oben genannten Parameter umgeschaltet oder durch Drücken oder Ziehen des Druck- oder Zugeingabeelements eine kontinuierliche Veränderung des Wertes vorgenommen werden.

Gemäß einer möglichen Ausgestaltung umfasst der Mikromanipulator einen Konnektor zur lösbaren Verbindung mit einem Operationsmikroskop.

Gemäß einer möglichen Ausgestaltung erfolgt die lösbar ausgeführte Befestigung des Mikromanipulators unter dem Operationsmikroskop mittels einer Schwalbenschwanz-Verbindung. Bei dem Konnektor handelt es sich daher um einen Schwalbenschwanz-Konnektor.

Gemäß einer bevorzugten Ausgestaltung erfolgt die lösbar ausgeführte Befestigung des Mikromanipulators unter dem Operationsmikroskop jedoch über einen Bajonettverschluss. Bei dem Konnektor handelt es sich daher um einen Bajonett-Konnektor. Insbesondere handelt es sich hierbei um einen Bajonettverschluss-Ring, welcher in eine Bajonettverschluss-Aufnahme am Operationsmikroskop einschiebbar und dort durch Drehen befestigbar ist. Anders als bei der bisher üblichen Verbindung mittels eines Schwalbenschwanz-Konnektors kann der Mikromanipulator hierdurch schnell an eine reproduzierbare Endposition gebracht werden.

Der Bajonettverschluss weist bevorzugt eine Raste auf, durch welche er in der Endposition gesichert ist. Das Einrasten kann durch Federn und/oder Kugeldruckstücke, ggf. unterstützt durch Schwerkraft, erfolgen. Die Raste, Federn und/oder Kugeldruckstücke sind bevorzugt auf dem am Operationsmikroskop angeordneten Konnektor des Bajonettverschluss vorgesehen.

Alternativ oder zusätzlich weist der Bajonettverschluss bevorzugt eine Arretierung auf, in welcher die Verbindung in der Endposition fixiert werden kann. Insbesondere kann hierfür eine Klemmschraube vorgesehen sein. Die Arretierung ist bevorzugt an dem am Mikromanipulator angeordneten Konnektor des Bajonett-Verschlusses vorgesehen.

Die beiden Konnektoren des Bajonettverschlusses werden bevorzugt in einer Richtung parallel zur Achse des Beobachtungsstrahlengangs des Mikroskops ineinander bzw. auseinandergeschoben und durch Drehen miteinander verbunden bzw. voneinander gelöst. Der Bajonettverschluss ist hierbei bevorzugt durch eine Drehbewegung, welche um eine parallel zur Achse des Beobachtungsstrahlengangs des Mikroskops verlaufende Drehachse verläuft, lösbar bzw. schließbar. Die Richtung der Anbringung (senkrecht von unten) ist damit entkoppelt von der Einrastrichtung (Rotation).

Die vorliegende Erfindung umfasst weiterhin ein Laserchirurgie-Gerät mit einem Mikromanipulator, wie er oben beschrieben wurde.

Gemäß einer möglichen Ausgestaltung der vorliegenden Erfindung umfasst das Laserchirurgie-Gerät einen Laser zur Erzeugung des Laserstrahls und/oder ein Operationsmikroskop, an welchem der Mikromanipulator lösbar befestigbar ist.

Sämtliche oben genannten, durch das Bedienelement oder die Bedienelemente angesteuerten Funktionen werden durch eine oder mehrere Steuerungen des Laserchirurgie-Geräts und/oder des Scanners bereitgestellt, welche entsprechend ausgestaltet sind, um eine entsprechende Ansteuerung insbesondere der Scanfigur vorzunehmen.

Die Steuerung oder Steuerungen umfasst insbesondere einen Mikroprozessor und einen nicht-flüchtigen Speicher, auf welchem eine Software abgespeichert ist, welche bei einem Ablaufen auf dem Microprozessor die entsprechenden Funktionen durchführt bzw. implementiert. Ein Eingang der Steuerung oder Steuerungen steht hierbei mit dem oder den Bedienelementen in Signalverbindung, ein Ausgang der Steuerung oder Steuerungen mit dem Laser und/oder einem Aktor des Scanners, insbesondere einem Aktor einer Spiegeleinheit des Scanners, über deren Bewegung die Scanfigur erzeugt wird.

Die vorliegende Erfindung wird nun anhand von Figuren und Zeichnungen näher beschrieben.

Dabei zeigen:
- Fig. 1: ein Ausführungsbeispiel eines erfindungsgemäßen Mikromanipulators in einer perspektivischen Ansicht,
- Fig. 2: das in Fig. 1 gezeigte Ausführungsbeispiel bei der Erzeugung einer Scanfigur,
- Fig. 3: das in Fig. 1 und 2 gezeigte Ausführungsbeispiel bei der Drehung einer Scanfigur, welche über die Drehung des Drehelements angesteuert wird,
- Fig. 4: das in Fig. 1 bis 3 gezeigte Ausführungsbeispiel, wobei ein Geometrieparameter der Scanfigur durch Betätigung eines Druck- oder Zugeingabeelements verändert wird, und
- Fig. 5: ein Ausführungsbeispiel eines erfindungsgemäßen Laser-Chirurgiegerätes mit einem erfindungsgemäßen Mikromanipulators in einer Prinzipdarstellung.

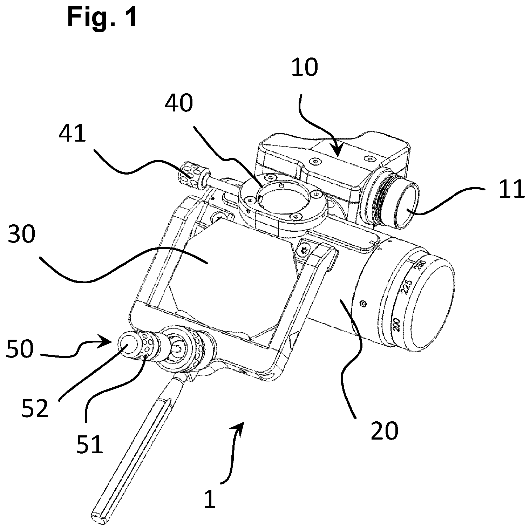

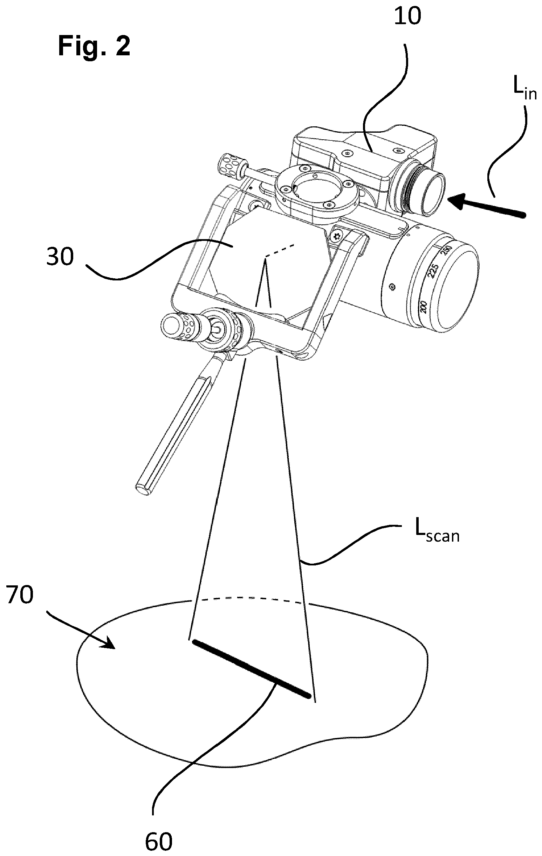

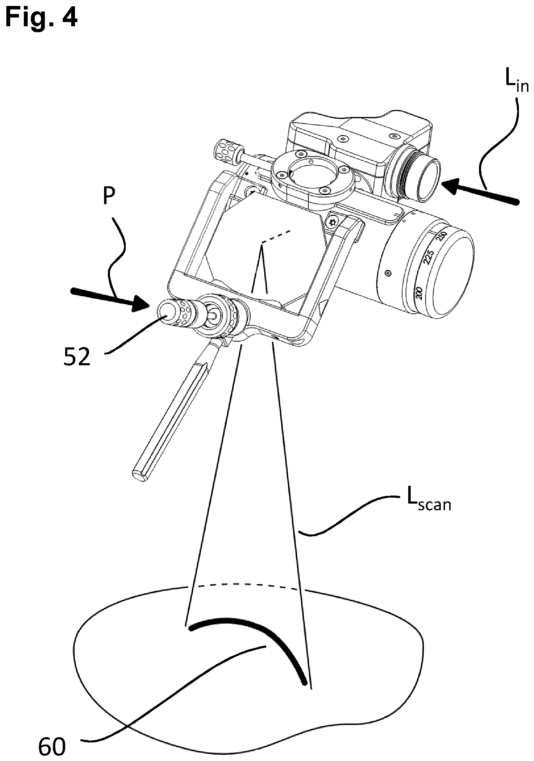

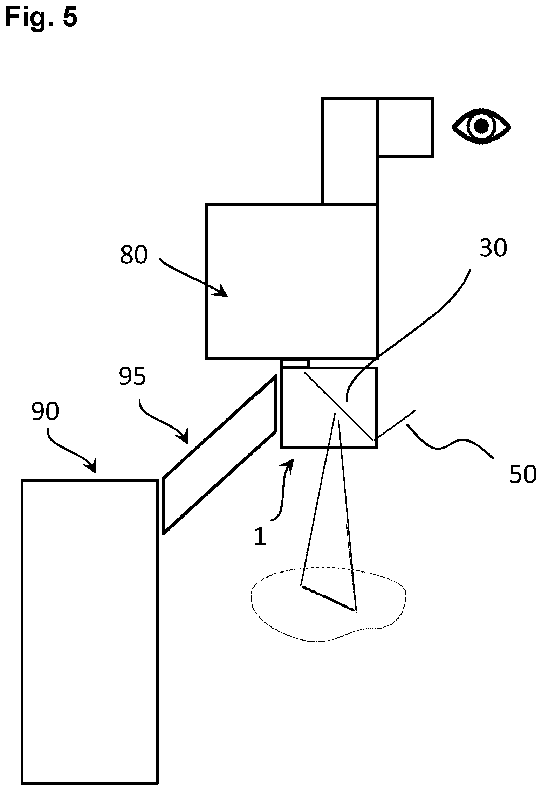

Fig. 1 bis 4 zeigen ein Ausführungsbeispiel eines erfindungsgemäßen Mikromanipulators, Fig. 5 dessen Einsatz in einem Laser-Chirurgiegerät, insbesondere für die mikrochirurgische Anwendung.

Für die mikrochirurgische Anwendung wird wie in Fig. 5 dargestellt die Beobachtung der Operation über ein Operationsmikroskop 80 vorgenommen, um auch sehr kleine Operationen adäquat durchführen zu können. Bei der Anwendung von Laserlicht für den chirurgischen Eingriff wird dieses über den Mikromanipulator 1 in den Beobachtungsstrahlengang des Operationsmikroskops 80 eingespiegelt.

Im Ausführungsbeispiel ist der Mikromanipulator 1 als eigenständiges Gerät ausgeführt, welches unter das Operationsmikroskop 80 montiert wird. Hierfür weist es einen Konnektor 40 auf.

Im Ausführungsbeispiel ist der Konnektor 40 am Mikromanipulator 1 als BajonettVerschluss-Ring ausgeführt, welcher mit einem entsprechenden Gegenelement am Operationsmikroskop durch Ineinanderschieben in einer Richtung parallel zur Achse des Beobachtungsstrahlengangs des Mikroskops und darauffolgendes Drehen miteinander verbindbar ist. Der Bajonettverschluss rastet in der Endposition ein. Weiterhin ist eine Klemmschraube 41 vorgesehen, durch welche der Bajonettverschluss in der Endposition fixiert werden kann. Eine Befestigung durch eine Schwalbenschwanzverbindung wäre jedoch ebenfalls denkbar.

Dem Mikromanipulator 1 wird über seinen optischen Eingang 11 ein Laserstrahl Lᵢₙ zugeführt. Dieser wird von einem Laser 90 erzeugt, bei welchem es sich beispielsweise um einen CO2-Laser, Festkörper- oder Dioden-Laser handelt. Der Laserstrahl wird über ein Rohr 95 von dem Laser 90 zu dem optischen Eingang 11 des Mikromanipulators 1 geführt. Um den zur Operation eingesetzten Laserstrahls sichtbar zu machen, ist weiterhin ein mit diesem kolinearer Pilotlaser vorgesehen.

Im Ausführungsbeispiel umfasst der Mikromanipulator 1 eine Fokussieroptik über welche der Laserstrahl Lᵢₙ fokussiert werden kann.

Der Mikromanipulator 1 umfasst weiterhin eine elektronisch gesteuerte Spiegeleinheit 10. Diese bewegt den Laserstrahl entlang einer Bahn. Durch die schnelle Strahlbewegung L_{scan} entsteht eine Strahlablenkungsstruktur, hier als Scanfigur 60 bezeichnet, auf dem Operationsfeld 70.

Im Ausführungsbeispiel umfasst der Mikromanipulator 1 weiterhin einen wellenlängenabhängigen Spiegel 30, welcher sich in der montierten Position des Mikromanipulators direkt unter dem Objektiv des Operationsmikroskops 80 befindet. Der Spiegel 30 ist durchlässig für die OP-Feldbeleuchtung und für die Beobachtung und hochreflektierend für das Laserlicht. Er steht unter ca. 45° vor dem Objektiv und reflektiert so den fokussierten Laserstrahl L_{scan} direkt in das Operationsfeld 70.

Der Mikromanipulator 1 umfasst einen Joystick 50, über welchen die Position des Laserstrahls im Operationsfeld und insbesondere die Position der Scanfigur einstellbar ist.

Im Ausführungsbeispiel handelt es sich bei dem Joystick 50 um ein mechanisches Element, welches den Spiegel 30 in 2 Achsen leicht kippen kann, um den Laserstrahl in seiner Position im Operationsbereich zu verändern.

Alternativ könnte der Joystick als ein elektronischer Joystick, wie er z.B. bei Computerspielen verwendet wird, ausgeführt sein, welcher elektrische Steuersignale erzeugt, über welche die Position des Laserstrahls im Operationsbereich eingestellt wird. Dieser kann wie in den Figuren gezeigt an der die optischen Komponenten enthaltenden Baueinheit angeordnet sein, wie dies von mechanischen Joysticks bekannt ist, oder an einer separaten, frei platzierbaren Konsole. Durch einen elektronischer Joystick ergeben sich auch in der Ansteuerung mehrere Alternativen:
In einer ersten Variante könnten die elektrischen Steuersignale an die Steuerung des bevorzugt separat zum Spiegel vorgesehenen Scanners 10 übertragen werden und die Nullposition des Scanners so ansteuern, dass das Zentrum der Scanfigur bewegt wird. Hierdurch könnte auf die bisher übliche separate Mechanik zum Bewegen des Spiegels 30 verzichtet werden und dieser fest angeordnet sein.

Alternativ könnten Mikroaktoren vorgesehen sein, welche durch den elektronischen Joystick angesteuert werden und den Spiegel 30 kippen und neigen.

In diesem Fall kann der Scanner 10 in den Spiegel integriert werden, indem die Mikroaktoren sowohl die Scanfunktion des Laserlichts als auch die Positionierung der Scanfigur übernehmen. Hierfür sind entsprechend schnelle Miktoaktoren notwendig.

Im Ausführungsbeispiel ist der Scanner 50 jedoch zusätzlich zu dem beweglichen Spiegel 50 vorgesehen und am optischen Eingang des Mikromanipulators 1 angeordnet.

Durch das Kippen des Spiegels 30 durch den Joystick 50 kann der Mittelpunkt der Scanfigur 60 im Operationsfeld 70 verschoben werden.

Insbesondere kann es sich bei der Scanfigur 60 zumindest in einem Betriebsmodus um eine Linie handeln. Diese besitzt eine Orientierung im Operationsfeld 70. Eine Linie wird verwendet, wenn die Operation, d.h. die Anwendung des Laserlichts einen Schnitt erzeugen soll. Durch die elektronische Steuerung des Scanners 10 erfolgt der Schnitt sehr gleichmäßig über die Linienlänge. Dieser Vorteil gegenüber einer handgeführten Linie wird in der Laseroperationstechnik zum präzisen Schneiden verwendet.

Stimmt die Orientierung der Linie nicht mit der gewünschten Schnittlinie überein, so muss die Scanfigur 60 gedreht werden. Auch bei anderen Scanfiguren kann eine Ausrichtung der Scanfigur notwendig sein.

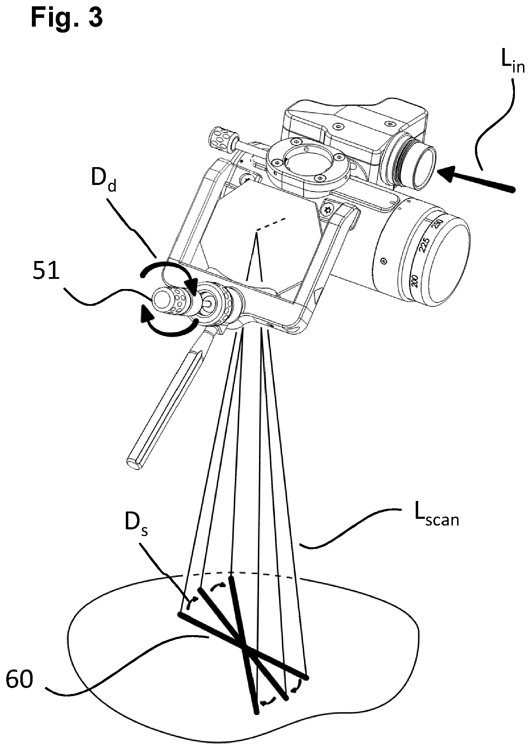

Hier setzt die Erfindung an, indem sie wie in Fig. 3 gezeigt durch ein Drehelement 51, welches im Ausführungsbeispiel am Joystick 50 des Mikromanipulators angeordnet ist, eine Drehung Dₛ der Scanfigur proportional zum Drehwinkel D_{d} des Drehelements 51 ermöglicht.

Im Ausführungsbeispiel ist das Drehelement 51 frei drehbar am Ende des Joysticks 50 angebracht.

Das Drehelement 51 befindet sich auf der Joystickachse befindet und ist axial fixiert. Es kann kräftefrei rotieren und bedient einen Drehgeber, welcher die relative Rotation D_{d} des Drehelements 51 um die Joystickachse in drehrichtungsabhängige, bevorzugt digitale Impulse umsetzt.

Das Drehelement 51 überträgt elektronisch seine Drehposition zur Steuerung des Scanners 10. Die Steuerung dreht die Scanfigur 60 entsprechend der Drehung des Drehelements 51.

Insbesondere werden die Impulse des Drehgebers im Ausführungsbeispiel über ein integriertes Kabel, welches unbewegt durch die Rotation des Drehelements 51 innerhalb des Joysticks 50 und des Mikromanipulators zur Signalweiterleitung an den Scanner 10 und/oder an den Laser 90 verläuft, weitergegeben.

Die direkte Drehwinkelverbindung von Drehelement 51 und Scanfigurendrehwinkel ermöglicht eine gezielte Drehung der Scanfigur 60. Die Drehungen des Drehelements sind nicht limitiert und in beide Drehrichtungen möglich.

Die Drehung Dₛ der Scanfigur 60 wird daher in Drehgeschwindigkeit und Richtung durch die Drehung D_{d} des Drehelements durch den Anwender bestimmt. Ebenfalls kann über das Drehelement eine Drehposition Dₛ angefahren werden, indem das Drehelement um einen frei bestimmten Drehwinkel D_{d} gedreht wird und die Scanfigur ebenfalls um diesen oder einen hierzu proportionalen Drehwinkel Dₛ gedreht wird. Zwischen Drehgeberwinkel und Figurendrehwinkel kann eine Unter- oder Übersetzung vorgesehen sein. Die Größe der Unter- oder Übersetzung kann auch dynamisch von der Drehgeschwindigkeit abhängig sein.

Die Erfindung ermöglicht daher eine kräftefreie Drehsteuerung der Scanfiguren ohne Limitierung des Gesamtdrehwinkels. Ein Intuitives Drehen wird durch Folgen der Figur beim Drehen des Drehelements ermöglicht.

Im Ausführungsbeispiel gibt es wie in Fig. 3 gezeigt weiterhin die Möglichkeit, das Drehelement 51 in Richtung der Joystickachse zu drücken, so dass dieses als Druckeingabeelement 52 fungiert. Alternativ könnte an der Spitze des Joysticks ein von dem Drehelement separates Druckeingabeelement 52 vorgesehen sein, welches von dem Drehelement 51 umgeben ist.

Der Druck P betätigt einen integrierten Drucktaster, welcher sein Signal ebenfalls an das Lasergerät übermittelt. Dies ermöglicht es, Parameter am Lasergerät und/oder Scanner hin- und herzuschalten oder stufenweise oder stufenlos Zyklen von Parametereinstellungen zu durchlaufen.

Insbesondere kann in einem Betriebsmodus oder einer Ausgestaltung wie in Fig. 3 gezeigt, die Linie, wenn sie als Scanfigur gewählt ist, durch Drücken des Druckeingabeelements 52 in ihrer Form verändert werden. Z. B. kann die Linie weniger und/oder stärker gekrümmt werden, z.B. um diese Schnittform dem zu schneidenden Gewebe anzupassen.

**In** einem weiteren Betriebsmodus oder einer weiteren Ausgestaltung ist vorgesehen, durch Drücken des Druckeingabeelements 52 die Übersetzung von Drehelementdrehwinkel D_{d} zu Figurendrehwinkel Dₛ zu verändern. Hierdurch wird der Drehgeber empfindlicher oder die Drehgeschwindigkeit der Scanfigur wird vergrößert. Beispielsweise kann die Übersetzung so eingestellt werden, dass 100° Drehwinkel des Drehelements z.B. nur 50° Drehwinkel der Scanfigur oder aber 200° bewirken.

**In** einem weiteren Betriebsmodus oder einer weiteren Ausgestaltung ist vorgesehen, durch Drücken des Druckeingabeelements 52 die Form der Scanfigur zu wechseln. Mögliche Formen sind Kreis, Linie, Quadrat, Rechteck, Dreieck oder Sechseck.

**In** einem weiteren Betriebsmodus oder einer weiteren Ausgestaltung ist vorgesehen, durch Drücken des Druckeingabeelements 52 die Stärke des Laserlichts zu verändern. Dies kann durch Änderung der Laserleistung oder auch durch die Geschwindigkeit des Scanvorgangs erreicht werden.

In einem weiteren Betriebsmodus oder einer weiteren Ausgestaltung ist vorgesehen, durch Drücken des Druckeingabeelements 52 die Helligkeit eines Pilotlaser einstellbar ist. Beispielsweise kann die Helligkeit durch mehrmaliges Drücken stufenweise einstellbar sein.

Gemäß einer möglichen Ausgestaltung ist durch Drücken des Druckeingabeelements 52 die Funktion des Drehelementes veränderbar. Insbesondere ist vorgesehen, dass durch Drücken des Druckeingabeelements 52 in einen Betriebsmodus geschaltet werden kann, in welchem durch Drehen des Drehelementes die Helligkeit des Pilotlasers einstellbar ist. Bevorzugt blinkt in diesem Betriebsmodus der Pilotlaser, um auf die Einstellbarkeit hinzuweisen. Das Umschalten der Funktion des Drehelementes kann insbesondere über ein langes Drücken des Druckeingabeelements 52 erfolgen.

Weiterhin ist denkbar, dass auch für die oben beschriebenen weiteren Funktionen, welche durch Drücken des Druckeingabeelements 52 angesteuert werden, entsprechende Betriebsmodi vorgesehen sind, welche durch Drücken des Druckeingabeelements 52 erreichbar sind und in welchen das Drehelement zur Ansteuerung der weiteren Funktionen dient.

Weiterhin besteht die Möglichkeit, neben oder anstelle des Druckeingabeelements 52 und insbesondere des Drucktaster im Drehelement ein Zugeingabeelements und insbesondere einen Zugtaster vorzusehen. Über diesen können die vorgenannten Funktionen ebenfalls angesteuert werden.

## Patentansprüche

1. Mikromanipulator (1) zur Manipulation eines Laserstrahls (Lᵢₙ, L_{scan}) eines Laserchirurgie-Geräts, mit einem Scanner (10) zur Erzeugung mindestens einer Scanfigur (60) und mindestens einem Bedienelement zur Ansteuerung des Scanners (10), wobei das Bedienelement ein Drehelement (51) umfasst, wobei eine Drehung (D_{d}) des Drehelements (51) eine Drehung (Dₛ) der Scanfigur (60) bewirkt, welche von der Größe und/oder Geschwindigkeit der Drehung (D_{d}) des Drehelements (51) abhängt,
**dadurch gekennzeichnet, dass** das Drehelement (51) ohne Begrenzung des Drehwinkels frei drehbar ist und die Scanfigur (60) dieser freien Drehung ohne Einschränkungen folgt.

2. Mikromanipulator (1) nach Anspruch 1, mit einem schwenkbar an einer Basis angeordneten Joystick (50), über welchen die Position der Scanfigur (60) einstellbar ist, wobei das Drehelement (51) an dem Joystick (50) angeordnet ist.

3. Mikromanipulator (1) nach Anspruch 2, wobei das Drehelement (51) um eine Hauptachse des Joysticks (50) drehbar ist und/oder wobei das Drehelement (51) im Bereich der Spitze des Joysticks (50) angeordnet ist, welche bevorzugt durch eine drehbare Spitze des Joysticks (50) gebildet wird.

4. Mikromanipulator (1) nach einem der vorangegangenen Ansprüche, wobei das Bedienelement einen Sensor umfasst, durch welchen eine absolute Drehwinkelposition und/oder eine Größe und/oder Geschwindigkeit einer relativen Veränderung der Drehwinkelposition des Drehelements (51) erfasst wird, wobei der Sensor bevorzugt die erfasste Größe als einen analogen oder digitalen Wert an eine Steuerung des Scanners (10) übermittelt.

5. Mikromanipulator (1) nach einem der vorangegangenen Ansprüche, wobei das Drehelement (51) stufenlos oder gestuft in eine Mehrzahl von Drehwinkelpositionen drehbar ist.

6. Mikromanipulator (1) nach einem der vorangegangenen Ansprüche, wobei das Drehelement (51) in zwei entgegengesetzte Drehrichtungen drehbar ist, wobei die Drehung (D_{d}) am Drehelement (51) eine gleichsinnige Drehung (Dₛ) der Scanfigur (60) bewirkt.

7. Mikromanipulator (1) nach einem der vorangegangenen Ansprüche, wobei die Drehung (D_{d}) des Drehelements (51) eine Drehung (Dₛ) der Scanfigur (60) bewirkt, welche von der Größe der Drehung (D_{d}) des Drehelements (51) abhängt, wobei die Drehung (D_{d}) bevorzugt eine zur Größe der Drehung proportionale Drehung (Dₛ) der durch den Scanner (10) erzeugten Scanfigur (60) bewirkt.

8. Mikromanipulator (1) nach einem der vorangegangenen Ansprüche, wobei zumindest in einem Bedienmodus die Größe der Drehung (Dₛ) der Scanfigur (60) ein festes Verhältnis zur Größe der Drehung (D_{d}) des Drehelements (51) besitzt.

9. Mikromanipulator (1) nach Anspruch 8, wobei das feste Verhältnis einstellbar ist.

10. Mikromanipulator (1) nach einem der vorangegangenen Ansprüche, wobei zumindest in einem Bedienmodus die Größe der Drehung (Dₛ) der Scanfigur (60) ein Verhältnis zur Größe der Drehung (D_{d}) des Drehelements (51) besitzt, welches von der Drehgeschwindigkeit der Drehung (D_{d}) des Drehelements (51) abhängt, wobei sich bevorzugt die Größe der Drehung (Dₛ) der Scanfigur (60) im Verhältnis zur Größe der Drehung (D_{d}) des Drehelements (51) bei steigender Drehgeschwindigkeit der Drehung (D_{d}) des Drehelements (51) erhöht.

11. Mikromanipulator (1) nach einem der vorangegangenen Ansprüche, wobei das Bedienelement ein Druck- oder Zugeingabeelement (52), insbesondere als Druck- oder Zugtaster oder Druck- oder Zugschalter, umfasst, wobei das Drehelement (51) bevorzugt als ein solches Druck- oder Zugeingabeelement (52) ausgestaltet ist oder ein solches Druck- oder Zugeingabeelement (52) aufweist, wobei durch Betätigen des Druck- oder Zugeingabeelements (52) mindestens ein Parameter des Mikromanipulators (1) veränderbar ist.

12. Mikromanipulator (1) nach Anspruch 11, wobei ein durch das Betätigen des Druck- oder Zugeingabeelements veränderbarer Parameter die Art und/oder Größe der Scanfigur (60) und/oder ein Geometrieparameter der Form einer gewählten Scanfigur (60), insbesondere die Krümmung einer linienförmigen Scanfigur (60), ist.

13. Mikromanipulator (1) nach Anspruch 11 oder 12, wobei ein durch das Betätigen des Druck- oder Zugeingabeelements veränderbarer Parameter die Laserleistung und/oder die Scanfigurengeschwindigkeit ist.

14. Mikromanipulator (1) nach einem der Ansprüche 11 bis 13, wobei ein durch das Betätigen des Druck- oder Zugeingabeelements veränderbarer Parameter der Bedienmodus des Drehelements (51) und/oder das Verhältnis zwischen der Größe der Drehung (Dₛ) der Scanfigur (60) und der Größe der Drehung (D_{d}) des Drehelements (51) ist.

15. Laserchirurgie-Gerät mit einem Mikromanipulator (1) nach einem der vorangegangenen Ansprüche, wobei das Laserchirurgie-Gerät bevorzugt einen Laser zur Erzeugung des Laserstrahls (Lᵢₙ, L_{scan}) und/oder ein Operationsmikroskop (80), an welchem der Mikromanipulator (1) lösbar befestigbar ist, umfasst.

## Claims

1. Micromanipulator (1) for manipulating a laser beam (Lᵢₙ, L_{scan}) of a laser surgery device, with a scanner (10) for generating at least one scan figure (60) and at least one control element for controlling the scanner (10), wherein the control element comprises a rotary element (51), wherein a rotation (D_{d}) of the rotary element (51) causes a rotation (Dₛ) of the scan figure (60) which depends on the magnitude and/or speed of the rotation (D_{d}) of the rotary element (51),
**characterized in that** the rotary element (51) is freely rotatable without limitation of the rotation angle and the scan figure (60) follows this free rotation without restrictions.

2. Micromanipulator (1) according to claim 1, with a joystick (50) arranged pivotably on a base, via which the position of the scan figure (60) can be set, wherein the rotary element (51) is arranged on the joystick (50).

3. Micromanipulator (1) according to claim 2, wherein the rotary element (51) is rotatable about a main axis of the joystick (50) and/or wherein the rotary element (51) is arranged in the region of the tip of the joystick (50), which is preferably formed by a rotatable tip of the joystick (50).

4. Micromanipulator (1) according to one of the preceding claims, wherein the control element comprises a sensor by means of which an absolute angular position and/or a magnitude and/or speed of a relative change in the angular position of the rotary element (51) is detected, wherein the sensor preferably transmits the detected magnitude as an analog or digital value to a control system of the scanner (10).

5. Micromanipulator (1) according to one of the preceding claims, wherein the rotary element (51) is rotatable continuously or in steps into a plurality of angular positions.

6. Micromanipulator (1) according to one of the preceding claims, wherein the rotary element (51) is rotatable in two opposite directions of rotation, wherein the rotation (D_{d}) at the rotary element (51) causes a rotation (Dₛ) of the scan figure (60) in the same sense.

7. Micromanipulator (1) according to one of the preceding claims, wherein the rotation (D_{d}) of the rotary element (51) causes a rotation (Dₛ) of the scan figure (60) which depends on the magnitude of the rotation (D_{d}) of the rotary element (51), wherein the rotation (D_{d}) preferably causes a rotation (Dₛ), proportional to the magnitude of the rotation, of the scan figure (60) generated by the scanner (10).

8. Micromanipulator (1) according to one of the preceding claims, wherein, at least in one operating mode, the magnitude of the rotation (Dₛ) of the scan figure (60) has a fixed ratio to the magnitude of the rotation (D_{d}) of the rotary element (51).

9. Micromanipulator (1) according to claim 8, wherein the fixed ratio is adjustable.

10. Micromanipulator (1) according to one of the preceding claims, wherein, at least in one operating mode, the magnitude of the rotation (Dₛ) of the scan figure (60) has a ratio to the magnitude of the rotation (D_{d}) of the rotary element (51) which depends on the rotational speed of the rotation (D_{d}) of the rotary element (51), wherein preferably the magnitude of the rotation (Dₛ) of the scan figure (60), relative to the magnitude of the rotation (D_{d}) of the rotary element (51), increases as the rotational speed of the rotation (D_{d}) of the rotary element (51) increases.

11. Micromanipulator (1) according to one of the preceding claims, wherein the control element comprises a push or pull input element (52), in particular as a push or pull button or push or pull switch, wherein the rotary element (51) is preferably configured as such a push or pull input element (52) or has such a push or pull input element (52), wherein by actuating the push or pull input element (52) at least one parameter of the micromanipulator (1) can be changed.

12. Micromanipulator (1) according to claim 11, wherein a parameter changeable by actuating the push or pull input element is the type and/or size of the scan figure (60) and/or a geometry parameter of the shape of a selected scan figure (60), in particular the curvature of a line-shaped scan figure (60).

13. Micromanipulator (1) according to claim 11 or 12, wherein a parameter changeable by actuating the push or pull input element is the laser power and/or the scan figure speed.

14. Micromanipulator (1) according to one of claims 11 to 13, wherein a parameter changeable by actuating the push or pull input element is the operating mode of the rotary element (51) and/or the ratio between the magnitude of the rotation (Dₛ) of the scan figure (60) and the magnitude of the rotation (D_{d}) of the rotary element (51).

15. Laser surgery device with a micromanipulator (1) according to one of the preceding claims, wherein the laser surgery device preferably comprises a laser for generating the laser beam (Lᵢₙ, L_{scan}) and/or a surgical microscope (80) on which the micromanipulator (1) can be detachably fastened.

## Revendications

1. Micromanipulateur (1) destiné à la manipulation d'un faisceau laser (Lᵢₙ, L_{scan}) d'un appareil de chirurgie laser, comprenant un scanner (10) pour générer au moins une figure de balayage (60) et au moins un organe de commande pour piloter le scanner (10), l'organe de commande comprenant un élément rotatif (51), une rotation (D_{d}) de l'élément rotatif (51) provoquant une rotation (Dₛ) de la figure de balayage (60), laquelle dépend de l'amplitude et/ou de la vitesse de la rotation (D_{d}) de l'élément rotatif (51),
**caractérisé en ce que** l'élément rotatif (51) est librement rotatif sans limitation de l'angle de rotation et que la figure de balayage (60) suit cette rotation libre sans restrictions.

2. Micromanipulateur (1) selon la revendication 1, comprenant un joystick (50) monté de manière pivotante sur une base, au moyen duquel la position de la figure de balayage (60) est réglable, l'élément rotatif (51) étant disposé sur le joystick (50).

3. Micromanipulateur (1) selon la revendication 2, dans lequel l'élément rotatif (51) peut tourner autour d'un axe principal du joystick (50) et/ou dans lequel l'élément rotatif (51) est disposé dans la zone de l'extrémité du joystick (50), laquelle est de préférence formée par une extrémité rotative du joystick (50).

4. Micromanipulateur (1) selon l'une quelconque des revendications précédentes, dans lequel l'organe de commande comprend un capteur au moyen duquel une position angulaire absolue et/ou une amplitude et/ou une vitesse d'une variation relative de la position angulaire de l'élément rotatif (51) est détectée, le capteur transmettant de préférence la grandeur détectée sous forme de valeur analogique ou numérique à une commande du scanner (10).

5. Micromanipulateur (1) selon l'une quelconque des revendications précédentes, dans lequel l'élément rotatif (51) peut être tourné en continu ou par crans dans une pluralité de positions angulaires.

6. Micromanipulateur (1) selon l'une quelconque des revendications précédentes, dans lequel l'élément rotatif (51) peut être tourné dans deux sens de rotation opposés, la rotation (D_{d}) de l'élément rotatif (51) provoquant une rotation (Dₛ) de même sens de la figure de balayage (60).

7. Micromanipulateur (1) selon l'une quelconque des revendications précédentes, dans lequel la rotation (D_{d}) de l'élément rotatif (51) provoque une rotation (Dₛ) de la figure de balayage (60), laquelle dépend de l'amplitude de la rotation (D_{d}) de l'élément rotatif (51), la rotation (D_{d}) provoquant de préférence une rotation (Dₛ) de la figure de balayage (60) générée par le scanner (10), proportionnelle à l'amplitude de la rotation.

8. Micromanipulateur (1) selon l'une quelconque des revendications précédentes, dans lequel, au moins dans un mode de fonctionnement, l'amplitude de la rotation (Dₛ) de la figure de balayage (60) présente un rapport fixe avec l'amplitude de la rotation (D_{d}) de l'élément rotatif (51).

9. Micromanipulateur (1) selon la revendication 8, dans lequel le rapport fixe est réglable.

10. Micromanipulateur (1) selon l'une quelconque des revendications précédentes, dans lequel, au moins dans un mode de fonctionnement, l'amplitude de la rotation (Dₛ) de la figure de balayage (60) présente un rapport avec l'amplitude de la rotation (D_{d}) de l'élément rotatif (51), lequel dépend de la vitesse de rotation de la rotation (D_{d}) de l'élément rotatif (51), l'amplitude de la rotation (Dₛ) de la figure de balayage (60) augmentant de préférence, par rapport à l'amplitude de la rotation (D_{d}) de l'élément rotatif (51), lorsque la vitesse de rotation de la rotation (D_{d}) de l'élément rotatif (51) augmente.

11. Micromanipulateur (1) selon l'une quelconque des revendications précédentes, dans lequel l'organe de commande comprend un élément d'entrée par pression ou par traction (52), notamment sous la forme d'un bouton-poussoir ou d'un bouton à tirer, ou d'un commutateur à pression ou à traction, l'élément rotatif (51) étant de préférence conçu comme un tel élément d'entrée par pression ou par traction (52) ou comportant un tel élément d'entrée par pression ou par traction (52), au moins un paramètre du micromanipulateur (1) pouvant être modifié par actionnement de l'élément d'entrée par pression ou par traction (52).

12. Micromanipulateur (1) selon la revendication 11, dans lequel un paramètre modifiable par actionnement de l'élément d'entrée par pression ou par traction est le type et/ou la dimension de la figure de balayage (60) et/ou un paramètre géométrique de la forme d'une figure de balayage (60) choisie, notamment la courbure d'une figure de balayage linéaire (60).

13. Micromanipulateur (1) selon la revendication 11 ou 12, dans lequel un paramètre modifiable par actionnement de l'élément d'entrée par pression ou par traction est la puissance laser et/ou la vitesse de la figure de balayage.

14. Micromanipulateur (1) selon l'une quelconque des revendications 11 à 13, dans lequel un paramètre modifiable par actionnement de l'élément d'entrée par pression ou par traction est le mode de fonctionnement de l'élément rotatif (51) et/ou le rapport entre l'amplitude de la rotation (Dₛ) de la figure de balayage (60) et l'amplitude de la rotation (D_{d}) de l'élément rotatif (51).

15. Appareil de chirurgie laser comprenant un micromanipulateur (1) selon l'une quelconque des revendications précédentes, l'appareil de chirurgie laser comprenant de préférence un laser pour générer le faisceau laser (Lᵢₙ, L_{scan}) et/ou un microscope opératoire (80), auquel le micromanipulateur (1) peut être fixé de manière amovible.
